# EUROPEAN PATENT APPLICATION

(11) **EP 1 854 458 A1**
(43) Date of publication of application: **14.11.2007**
(21) Application number: 06450071.3
(22) Date of filing: 08.05.2006
(51) Int. Cl.: A61K 31/00, A61P 37/00, A61P 17/00, A61K 38/20, A61K 38/16

(54) **Use of a compound with RANKL activity**

(71) Applicant: IMBA-Institut für Molekulare Biotechnologie GmbH, 1030 Vienna (AT)
(72) Inventor: Penninger, Josef, 2503 Baden bei Wien (AT); Loser, Karin, 48341 Altenberge (DE); Beissert, Stefan, 48143 Münster (DE)
(74) Representative: Sonn & Partner Patentanwälte

(57) **Abstract**

The present invention relates to the use of a compound with receptor activator of nuclear factor-KB ligand (RANKL) activity for the manufacture of a topical pharmaceutical formulation for the treatment or the prevention of skin-associated diseases.

## Description

The present invention relates to pharmaceutical preparations for the treatment or the prevention of skin-associated diseases.

Inflammation is a significant component in a number of skin disorders or diseases including, but not limited to, acne and rosacea, atopic dermatitis, contact dermatitis, drug eruptions, psoriasis, seborrheic dermatitis, connective tissue diseases (such as lupus, scleroderma, and rheumatoid arthritis), other autoimmune disorders such as the blistering disease bullous pemphigoid or pemphigus, pigmentary diseases (such as post inflammatory hyperpigmentation, melasma and vitiligo), urticaria or hives, inflammation associated with skin infections such as tinea corporis or fungal infection of the finger or toenails, among others. Modulating the inflammatory response has been shown to result in dramatic improvement in the conditions listed above. Standard treatment involves the use of topical corticosteroids, oral corticosteroids and other agents that modulate inflammation. However, topical corticosteroids have undesirable side effects such as skin atrophy, telangiectasia and the possibility of adrenal axis suppression thus limiting their long-term use. Significant research has been conducted in the field of radiation therapy to assess the ability of free-radical scavengers in protecting normal tissue from ionizing radiation. Sulfhydryl compounds were among the first radioprotectors to be identified. Their protective mechanism appears to be due to their ability to scavenge radiation-induced free radicals and/or donate reducing equivalents to oxidized molecules. Hematopoietic cytokines have also been investigated as radioprotectors. They are believed to protect by more quickly restoring hematopoietic function after radiation exposure.

It is an object of the present invention to provide pharmaceutical preparations which are used for the treatment of inflammatory diseases and/or skin-associated diseases avoiding the side effects regularly occurring with known medicaments like cortisone.

Therefore the present invention relates to the use of a compound with receptor activator of nuclear factor-κB ligand (RANKL) activity for the manufacture of a topical pharmaceutical formulation for the treatment or the prevention of inflammatory diseases.

Compounds exhibiting RANKL activity, especially RANKL itself (see e.g. US 6,419,929), are known to be involved in a series of bone related diseases like osteoporosis, rheumatoid arthritis, cancer-induced bone destruction, metastasis, hypercalcemia or pain (see e.g. Hofbauer, LC, et al. (2001) Cancer 92: 460-470). In addition to being important in bone biology, RANKL plays a role in the immune system by regulating antigen-specific T cell responses (see e.g. Anderson et al. (1997) Nature 390:175-9).

Regulatory CD4⁺CD25⁺ T cells play an important role in suppressing immune responses. The requirements for the homeostasis of peripheral CD4⁺CD25⁺ T cell remain incompletely understood. RANKL (also known as OPGL, TRANCE, or ODF) and its receptor RANK are key regulators of bone remodeling and formation of a lactating mammary gland. Furthermore, RANK-RANKL interactions are involved in lymph node formation and T cell/dendritic cell communication.

It was surprisingly found that RANKL is expressed in keratinocytes of the inflamed or UV exposed skin. RANKL over-expression in keratinocytes results in functional alterations of dendritic cells in the epidermis and draining lymph nodes and systemic increases of regulatory CD4⁺CD25⁺ T cells. Cutaneous antigen-presenting cells were found to be responsible for the peripheral expansion of CD4⁺CD25⁺ T cells. Thus, RANKL expression in the skin can change dendritic cell functions to control the peripheral homeostasis of CD4⁺CD25⁺ regulatory T cells. Importantly, regulatory T cells from epidermal RANKL transgenic mice suppressed allergic contact hypersensitivity responses and the development of systemic autoimmunity. Environmental stimuli at the skin can rewire the local and systemic immune system via RANKL.

Preferred compounds with RANKL activities according to the present invention are recombinantly produced RANKL molecules, e.g. produced by an expression system wherein the RANKL gene (at least the RANKL coding part thereof) has been introduced or by an activation of wt RANKL by transgenic gene expression elements (promoters, enhancers, etc.).

Compounds exhibiting RANKL activity may be experimentally tested and validated using in vivo and in vitro assays. Suitable assays include, but are not limited to, activity assays and binding assays. For example, RANKL activity assays, such as the tartrate resistant acid phosphatase assay (TRAP; Matsuzaki et al., Biochemical and biophysical research communications 246, 199-204 (1998)) for monitoring the differentiation of pre-osteoclast or RAW264.7 cells into osteoclasts, and the NF-.kappa.B (Wei et al., Endocrinology 142, 1290-I95, (2001)) or c-Jun (Srivastava et al., JBC 276, 8836-8840 (2001)) or NFATc1 (Takayanagi H. et al., Dev. Cell. 3, 889-901 (2002)) transcription factor activation assays for monitoring signaling through RANK are screens that may be utilized in identifying compound exhibiting RANKL activity. Other biological markers for osteoclasto-genesis include counting multinucleated TRAP staining cells, calcitonin receptor expression, the presence of ruffled borders on osteoclasts, and cathepsin K expression and activity (see Suda et al., Modulation of Osteoclast Differentiation and Function by the New Members of the Tumor Necrosis Factor Receptor and Ligand Families; Endocrine Reviews 20(3):345-357 (1999) and Garnero et al., The collagenolytic activity of cathepsin K is unique among mammalian proteinases; Journal of biochemistry 273(48):32347-32352 (1998)).

The compound according to the present invention may be also"fragments, derivatives or analogs" of RANKL which may also be used in the formulation according to the present invention exhibit similar if even not identical binding behaviour to RANK and/or OPG (osteoprotegerin) as naturally occurring RANKL. This applies also for RANKL fragments which have to comprise at least those parts of the protein which are responsible for the binding to RANK. For example, RANKL fragments, derivatives or analogs, which could be used as treatments for a variety of bone diseases, have been described e.g. in the US 5,843,678. In the WO 00/15807, for instance, RANKL variants are disclosed, which induce production of an immune response that down-regulates RANKL activity. Further RANKL variants are disclosed in the WO 03/059281, WO 03/033663, US 2003/219864 and US 2004/167072 A. It is of course possible to use RANKL or RANKL variants in a pharmaceutical formulation which are isolated from natural sources or synthesized chemically. The RANKL variants may also be produced recombinantely.

The inflammatory diseases according to the present invention may preferably be local or systemic inflammations.

It surprisingly turned out that the topical administration of RANKL does not only exhibit effects at the site of administration but has also systemic effects. Therefore, the topical administration of RANKL allows not only to treat or prevent local inflammations like psoriasis but also systemic inflammations like multiple sclerosis, atherosclerosis, arthritis, etc. Due to the topical administration of RANKL dendritic cells, e.g. are activated which consequently activate systemically regulatory T cells (Tregs) required for the treatment of prevention of systemic inflammatory diseases. According to a preferred embodiment of the present invention the inflammatory diseases are allergies, in particular contact allergies, and/or autoimmune diseases.

It surprisingly turned out that RANKL formulated in a topical pharmaceutical preparation may be employed to treat in particular inflammatory diseases which are the result of allergic reactions and autoimmune diseases wherein all of these diseases may be local or systemic.

The inflammatory diseases are preferably viral or bacterial inflammations or other inflammations.

In particular inflammations caused by viruses (e.g. herpes simplex viruses) and by exposure to chemicals and radiation (e.g. radioactive radiation) may be treated with the pharmaceutical preparation according to the present invention.

According to a preferred embodiment of the present invention the inflammatory diseases are skin-associated diseases, in particular skin-associated diseases selected from the group consisting of psoriasis, atopic dermatitis, alopecia areata, alopecia totalis, alopecia subtotalis, alopecia universalis, alopecia diffusa, lichen planus, dermatomyostis of the skin, atopic eczema, morphea, sklerodermia, psoriasis vulgaris, psoriasis capitis, psoriasis guttata, psoriasis inversa, alopecia areata ophiasis-type, androgenetic alopecia, allergic contact eczema, irritative contact eczema, contact eczema, pemphigus vulgaris, pemphigus foliaceus, pemphigus vegetans, scarring mucosal pemphigoid, bullous pemphgoid, mucous pemphigoid, dermatitis, dermatitis herpetiformis duhring, urticaria, necrobiosis lipoidica, erythema nodosum, lichen vidal, prurigo simplex, prurigo nodularis, prurigo acuta, linear IgA dermatosis, polymorphic light dermatoses, erythema solaris, lichen sclerosus et atrophicans, exanthema of the skin, drug exanthema, purpura chronica progressiva, dihidrotic eczema, Eczema, fixed drug exanthema, photoallergic skin reaction, lichen, simplex eriorale, dermatitis, "Graft versus Host-Disease", acne, rosacea, abnormal scarring, keloids, actinic keratosis, hyperkeratosis, epidermolytic hyperkeratosis, hyperkeratosis lenticularis perstans, keratosis pilaris, ichthyoses and vitiligo. Especially preferred diseases to be treated according to the present invention are diseases which, for instance, require a reduced exposure to UV radiation (e.g. sunlight) like lupus.

According to another preferred embodiment of the present invention the inflammatory diseases are selected from the group consisting of rheumatoid athritis, multiple sclerosis, type I diabetes, Hashimoto's disease, myokarditis, atherosclerosis, glomerulonephritis, autoimmune hepatitis, inflammatory Bowel disease and other inflammatory and autoimmune diseases.

The pharmaceutical formulation according to the present invention which comprises an effective amount of a compound with RANKL activity is preferably an ointment, a gel, a lotion, a foam, an emulsion, a liposome, a transferosome, a cream or a paste.

The compound according to the present invention can be formulated in any known topical pharmaceutical preparation listed above. Methods for formulating said pharmaceutical preparations are known to the person skilled in the art.

The skin as permeable barrier is mostly impermeable to molecules having molecular weight greater than about 750 Da. Hence, in order to let larger molecules, in particular proteins like RANKL and variants thereof, cross the skin, mechanisms other than normal osmosis must be used. Consequently, the pharmaceutical formulation according to the present invention has to comprise substances (e.g. carriers, excipients) which facilitate the transport of proteins through the skin to the epidermis and the dermis.

The pharmaceutical formulations of the present invention, which may be presented in unit dosage form, may be prepared according to conventional techniques well known in the art. Such techniques comprise bringing into association the active ingredients with one or more pharmaceutical carriers or excipients. In general the formulations are prepared by uniformly and intimately bringing into association the active ingredients with liquid carriers or finely divided solid carriers or both.

The compositions of the present invention may be formulated as suspensions in aqueous media or as aqueous solutions (lotion). Aqueous suspensions and solutions may further contain substances which increase the viscosity of the suspension including, for instance, sodium carboxymethylcellulose, sorbitol and/or dextran. The addition of the latter substances leads to the formation of a gel. The suspension and solution may also contain stabilizers (e.g. proteases).

In another embodiment of the present invention the pharmaceutical formulations may be formulated and used as foams. Pharmaceutical foams include formulations such as emulsions, creams and liposomes. While basically similar in nature these formulations vary in the components and the consistency of the final product. The know-how on the preparation of such compositions and formulations is generally familiar to those skilled in the art and may be applied to the formulation of the compositions of the present invention.

The compositions of the present invention may be prepared and formulated as emulsions. Emulsions are heterogenous systems of one liquid dispersed in another in the form of droplets ("Pharmaceutical Dosage Forms", Lieberman, Rieger and Banker (Eds.), volumes 1 to 3, Marcel Dekker (Publisher), 2nd volume 1998). In general, emulsions may be either of the water in oil (w/o) or of the oil in water (o/w) variety. Emulsions may contain additional components in addition to the dispersed phases and the active drug which may be present as a solution in either the aqueous phase, oily phase or as a separate phase itself. Pharmaceutical excipients such as emulsifiers, stabilizers, dyes and anti-oxidants may also be present in emulsions as needed. Pharmaceutical emulsions may also be multiple emulsions that are comprised of more than two phases such as, for example, in the case of oil in water in oil (o/w/o) and water in oil in water (w/o/w) emulsions. The aqueous phase may preferably be water, an aqueous solution of the drug, glycerol, PEG300, PEG400, polyglycerols, propylene glycols, and derivatives of ethylene glycol. The oil phase may include fatty acid esters, medium chain (C8-C12) mono, di, and tri-glycerides, polyoxyethylated glyceryl fatty acid esters, fatty alcohols, polyglycolized glycerides, saturated polyglycolized C8-C10 glycerides, vegetable oils and silicone oil. Emulsifiers preferably used in an emulsion according to the present invention may be synthetic surfactants, naturally occurring emulsifiers, absorption bases and finely dispersed solids ("Pharmaceutical Dosage Forms", Lieberman, Rieger and Banker (Eds.), volumes 1 to 3, Marcel Dekker (Publisher), 2nd volume 1998). Synthetic surfactants (surface active agents) are typically amphiphilic and comprise a hydrophilic and a hydrophobic portion. Furthermore the surfactants preferably employed may be of nonionic, anionic, cationic and amphoteric nature. Naturally occurring emulsifiers used in emulsion formulations include lanolin, beeswax, phosphatides, lecithin and acacia. A large variety of non-emulsifying materials are also included in emulsion formulations and contribute to the properties of emulsions. These include fats, oils, waxes, fatty acids, fatty alcohols, fatty esters, humectants, hydrophilic colloids, preservatives and antioxidants. Hydrophilic colloids or hydrocolloids include naturally occurring gums and synthetic polymers such as polysaccharides (for example acacia, agar, alginic acid, carrageenan, guar gum, karaya gum and tragacanth), cellulose derivatives (for example carboxymethylic cellulose and carboxypropyl cellulose) and synthetic polymers (for example carbomers, cellulose ethers and carboxyvinyl polymers). These disperse or swell in water to form colloidal solutions that stabilize emulsions by forming strong interfacial films around the dispersed-phase droplets and by increasing the viscosity of the external phase.

The pharmaceutical formulations according to the present invention may further comprise preservatives like methylparaben, propylparaben, quaternary ammonium salts, benzalkonium chloride, esters of p-hydroxybenzoic acid, boric acid and phenoxyethanol. The total amount of preservative will depend on the dosage form used and may in general be from about 0.1% to 20% by weight. In topical emulsion compositions according to the present invention, for instance, the preservative combination will be present in an amount from about 0.1% to 10%, preferably 0.5% to 8% and more preferably 1% to 5%. In a preferred embodiment, methylparaben and propylparaben may be present in an amount from about 0.1% to 1% and phenoxyethanol in an amount from about 1 to 5%.

Antioxidants are also commonly added to topical formulations to prevent deterioration of the formulation. Antioxidants used may be free radical scavengers such as tocopherols, alkyl gallates, butylated hydroxyanisole, butylated hydroxytoluene or reducing agents such as ascorbic acid and sodium metabisulfite and antioxidant synergists such as citric acid, tartaric acid and lecithin.

Vesicles, such as liposomes, have attracted great interest because of their specificity and the duration of action they offer from the standpoint of drug delivery. As used in the present invention, the term "liposome" means a vesicle composed of amphiphilic lipids arranged in a spherical bilayer or bilayers. Liposomes are unilamellar or multilamellar vesicles which have a membrane formed from a lipophilic material and an aqueous interior. The aqueous portion contains the composition to be delivered.

Liposomal formulations have been the focus of extensive investigation as the mode of delivery for many drugs. There is growing evidence that for topical administration, liposomes present several advantages over other formulations. Such advantages include reduced side-effects related to high systemic absorption of the administered drug, increased accumulation of the administered drug at the desired target, and the ability to administer a wide variety of drugs, both hydrophilic and hydrophobic, into the skin.

Neutral liposome compositions, for example, can be formed from dimyristoyl phosphatidylcholine (DMPC) or dipalmitoyl phosphatidylcholine (DPPC). Anionic liposome compositions may be formed from dimyristoyl phosphatidylglycerol, while anionic fusogenic liposomes are formed primarily from dioleoyl phosphatidylethanolamine (DOPE). Another type of liposomal composition is formed from phosphatidylcholine (PC) such as, for example, soybean PC, and egg PC. Another type is formed from mixtures of phospholipid and/or phosphatidylcholine and/or cholesterol.

Transfersomes are yet another type of liposomes, and are highly deformable lipid aggregates which are attractive candidates for drug delivery vehicles. Transfersomes may be described as lipid droplets which are so highly deformable that they are easily able to penetrate through pores which are smaller than the droplet. Transfersomes are adaptable to the environment in which they are used, e.g. they are self-optimizing (adaptive to the shape of pores in the skin), self-repairing, frequently reach their targets without fragmenting, and often self-loading. To make transfersomes it is possible to add surface edge-activators, usually surfactants, to a standard liposomal composition. Transfersomes have been used to deliver serum albumin to the skin. The transfersome-mediated delivery of serum albumin has been shown to be as effective as subcutaneous injection of a solution containing serum albumin.

Surfactants preferably used according to the present invention comprise nonionic surfactants which include nonionic esters such as ethylene glycol esters, propylene glycol esters, glyceryl esters, polyglyceryl esters, sorbitan esters, sucrose esters, and ethoxylated esters. Nonionic alkanolamides and ethers such as fatty alcohol ethoxylates, propoxylated alcohols, and ethoxylated/ propoxylated block polymers are also included in this class. Anionic surfactants include carboxylates such as soaps, acyl lactylates, acyl amides of amino acids, esters of sulfuric acid such as alkyl sulfates and ethoxylated alkyl sulfates, sulfonates such as alkyl benzene sulfonates, acyl isothionates, acyl taurates and sulfosuccinates, and phosphates. Cationic surfactants include quaternary ammonium salts and ethoxylated amines. Amphoteric surfactants include acrylic acid derivatives, substituted alkylamides, N-alkylbetaines and phosphatides. The use of surfactants in drug products, formulations and in emulsions has been reviewed (Rieger, in "Pharmaceutical Dosage Forms," Marcel Dekker, Inc., New York, N.Y., 1988, p. 285).

Ointments, creams or pastes according to the present invention are semi-solid formulations of the active ingredient for external application. They may be made by mixing the active ingredient in finely-divided or powdered form, alone or in solution or suspension in an aqueous or non-aqueous fluid, with the aid of suitable machinery, with a greasy or non-greasy base. The base may comprise hydrocarbons such as hard, soft or liquid paraffin, glycerol, beeswax, a metallic soap; a mucilage; an oil of natural origin such as almond, corn, arachis, castor or olive oil; wool fat or its derivatives or a fatty acid such as steric or oleic acid together with an alcohol such as propylene glycol or a macrogel. The formulation may incorporate any suitable surface active agent such as an anionic, cationic or non-ionic surfactant such as a sorbitan ester or a polyoxyethylene derivative thereof.

According to a preferred embodiment of the present invention the compound with RANKL activity is comprised in the formulation in a concentration between 0,0001 to 1 % w/w, preferably between 0,001 to 0,5 % w/w, more preferably between 0,001 to 0,05 % w/w. The compound according to the present invention may be preferably comprised in said formulation in a concentration of 1 ng/ml to 10 µg/ml, 10 ng/ml to 500 ng/ml or 10 ng/ml to 100 ng/ml.

According to another preferred embodiment of the present invention the formulation further comprises the formulation further comprises at least one compound selected from the group consisting of cortisone, interleukins, in particular IL-1, IL-6 and IL-17 tumor necrosis factor α, prostaglandin E2 and vitamin D3.

RANKL and compounds with RANKL activity can be applied topically also in combination with any other drug suitable to treat a skin disorder according to the present invention.

Another aspect of the present invention relates to a topical pharmaceutical formulation comprising receptor activator of nuclear factor-κB ligand (RANKL).

The pharmaceutical formulation according to this aspect of the present invention may comprise RANKL or said compound and components as outlined above.

The formulation is preferably an ointment, a gel, a lotion, a foam, an emulsion, a liposome, a transferosome, a cream, a paste or a patch.

The use of a patch as provided by the present invention can be advantageous in alleviating many of the problems associated with topical formulations as known in the prior art. For instance, the application time for a patch can be reduced compared to other topical compositions and also the penetration of the drug molecule through the skin is faster than with other conventional topical dosage forms. These features may lead to an increase of the bioavailability of the drug and, hence, to a better therapeutic efficacy.

A patch to be used according to the present invention may comprise a spray-on patch, a transdermal patch, a physical patch or a controlled release patch.

Patches preferably used according to the present invention are disclosed, for instance, in the US 6,455,066.

According to a preferred embodiment of the present invention the patch comprises the compound with RANKL activity in an amount of 0,0001 to 1 % w/w, preferably between 0,001 to 0,5 % w/w, more preferably between 0,001 to 0,05 % w/w.

The patch according to the present invention further comprises preferably at least one compound selected form the group consisting of cortisone, interleukins, in particular IL-1, IL-6 and IL-17, tumor necrosis factor α, prostaglandin E2 and vitamin D3.

The present invention is further illustrated by the following figures and examples, without being restricted thereto.

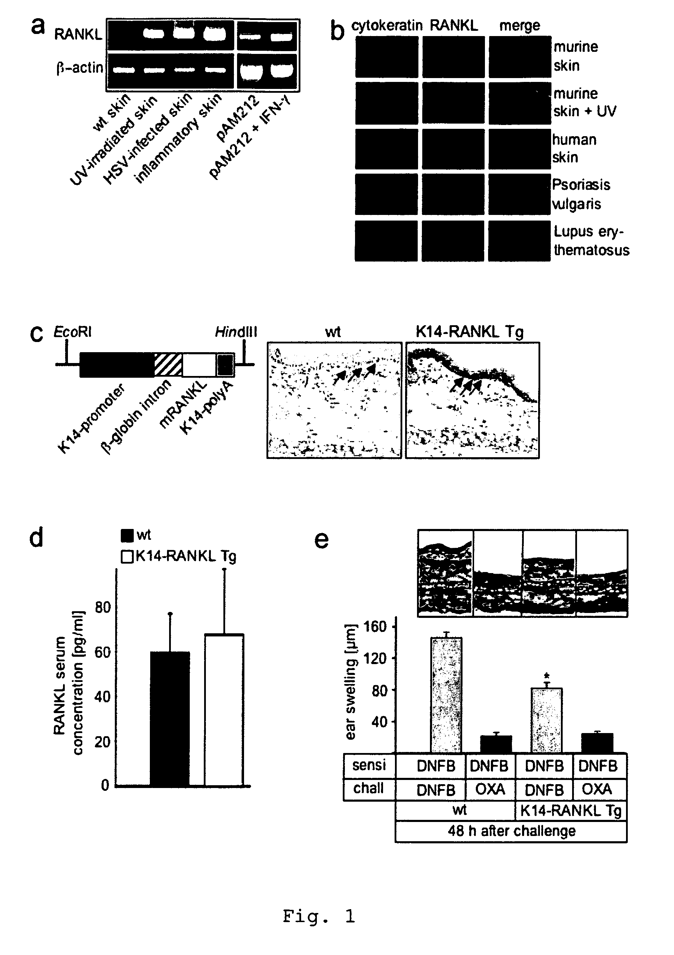

**Fig. 1.** Epidermal RANKL over-expression suppresses cutaneous contact hypersensitivity responses.
   a, RANKL is expressed in inflamed skin. Reverse transcriptase-PCR analysis of untreated-, ultraviolet B irradiated-(800 mJ/m²), and Herpes simplex virus type 1 infected-skin (18) from wild type mice, inflamed skin from CD40L transgenic mice, PAM212 keratinocytes, and PAM212 cells after stimulation with 50ng/ml IFN-γ.
   b, RANKL is expressed in murine and human keratinocytes and upregulated by UV irradiation or during inflammation.
      Immonofluorescence stainings of murine and human skin as well as lesional skin from Psoriasis vulgaris and Lupus erythematosus patients using antibodies directed against cytokeratin and RANKL. Original magnification: 200X.
   c, K14-RANKL Tg expression in basal keratinocytes. The construct contains the human K14-promoter, β-globin intron, mRANKL cDNA, and the K14-polyadenylation site (polyA). Immunhistochemical staining of ear skin using an antibody directed against murine RANKL. Basal keratinocytes are indicated by arrows. Original magnification: 200X.
   d, RANKL concentration in the serum of wild type (wt) and K14-RANKL Tg mice. RANKL levels were analyzed by ELISA. Data are shown as mean ± SD from five mice for each group.
   e, Reduced CHS responses in K14-RANKL Tg mice. Animals were sensitized with DNFB and DNFB ear challenged or sensitized with DNFB and challenged with oxazolone. Data are shown as mean ear swelling ± SD and are representative of 15 mice in three independent experiments. Representative H&E histologies of ear swelling are shown for each genotype. Magnifications X 200. The * indicates statistical significance (Student's t-test; p < 0.05).

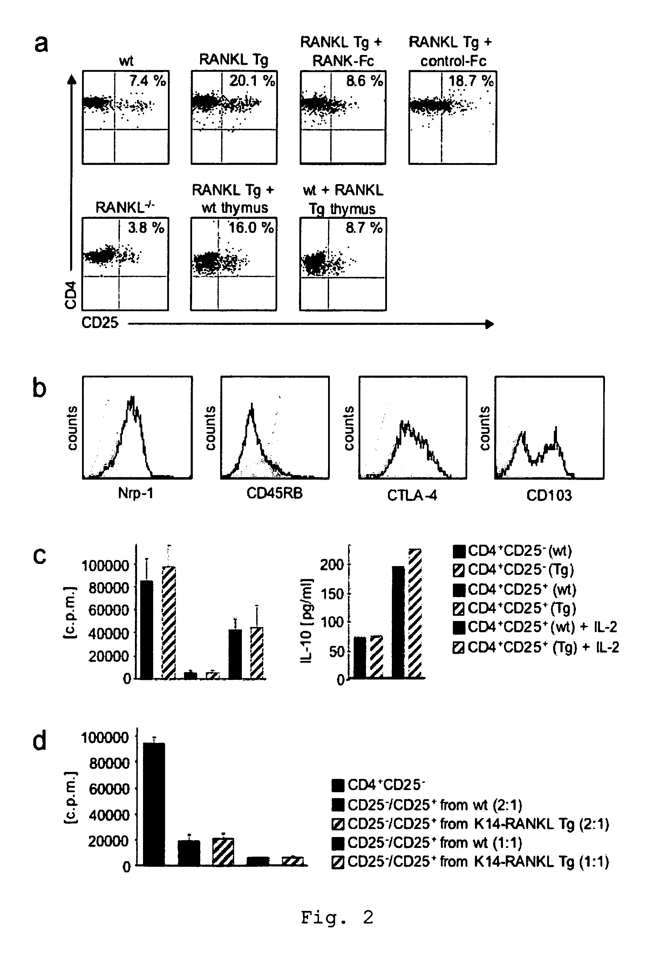

**Fig. 2.** Epidermal RANKL controls homeostasis of CD4⁺CD25⁺ regulatory T cells.
   a, RANKL expression in the skin induces expansion of CD4⁺CD25⁺ T cells. Flow cytometric analyses of splenic T cells from wild type (wt; total number of animals analyzed n=70), K14-RANKL Tg (n=50), K14-RANKL Tg mice treated with RANK-Fc (n=10), K14-RANKL Tg mice treated with control-Fc (n =5), rankl^{-/-} mice (n=5), thymectomized K14-RANKL Tg mice grafted with a wild type thymus (n=8), and thymectomized wt mice grafted with thymic tissue from K14-RANKL Tg mice (n=8). After 8-10 weeks, T cells from lymph nodes and spleens were analyzed for CD4 and CD25 expression by flow cytometry. Representative dot blots are shown for each experimental group.
   b, CD4⁺CD25⁺ T cells from K14-RANKL Tg mice show surface marker expression characteristic for regulatory T cells. CD4⁺CD25⁻ (grey) and CD4⁺CD25⁺ T cells (black lines) from spleens of K14-RANKL Tg mice were analyzed by flow cytometry. CTLA-4 staining was performed after cell permeabilization.
   c, CD4⁺CD25⁺ T cells display proliferative anergy and secrete IL-10. CD4⁺CD25⁻ and CD4⁺CD25⁺ T cells were separated by MACS and proliferation assays were performed by stimulating 2x10⁵ cells with anti-CD3 plus anti-CD28 with or without recombinant mIL-2 (20 Units/ml). For cytokine production, CD4⁺CD25⁻ and CD4⁺CD25⁺ T cells (5x10⁴) were stimulated with anti-CD3 and anti-CD28 and IL-10 levels were analyzed using the cytometric bead assay inflammation kit. Data show one out of three different experiments. * indicates statistical significance (Student's t-test; p < 0.05).
   d, CD4⁺CD25⁺ T cells from K14-RANKL Tg mice are immunosuppressive. CD4⁺CD25⁻ and CD4⁺CD25⁺ T cells were separated by MACS and proliferation assays performed by stimulating wild type CD4⁺CD25⁻ T cells (2x10⁵) with anti-CD3 and anti-CD28 Abs in the absence or presence of increasing numbers of CD4⁺CD25⁺ T cells from wt or K14-RANKL Tg mice. Mean values of 3H-thymidine uptake +/- SD are shown from one out of three experiments. * indicates statistical significance (Student's t-test; p < 0.05).

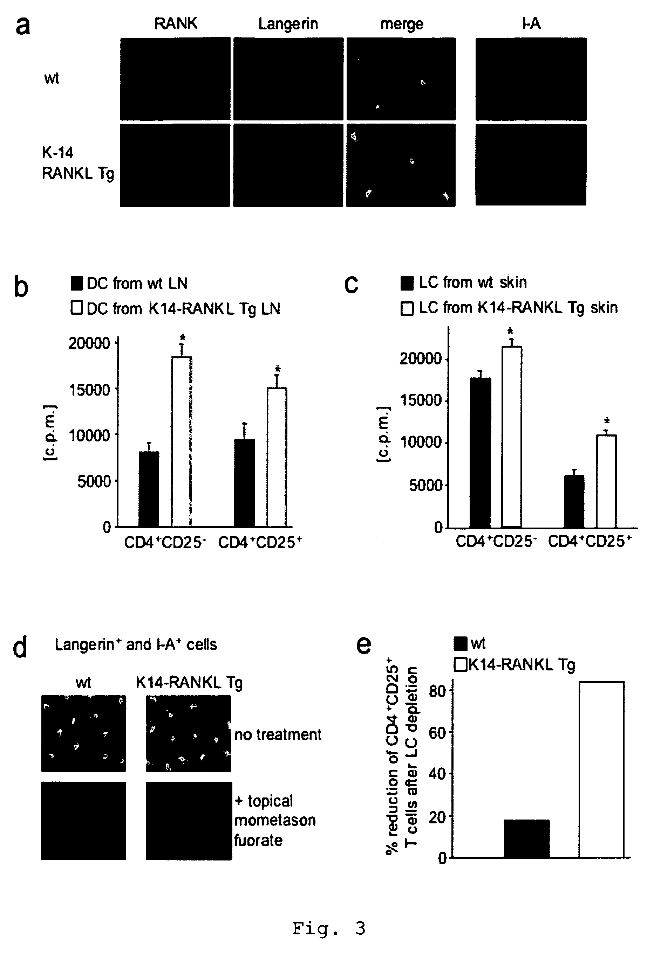

**Fig. 3.** Epidermal Langerhans cells control systemic homeostasis of CD4⁺CD25⁺ regulatory T cells.
   a, K14-RANKL Tg mice have normal numbers of epidermal LCs expressing RANK, Langerin, and MHC class II I-A molecules. Ear sheets of wild type and K14-RANKL Tg mice were stained with Abs to the indicated molecules. Merged images for Langerin and RANK are shown in yellow (Magnification X 400).
   b-c, Lymph node DCs (b) and epidermal LCs (c) from K14-RANKL Tg mice have an increased capacity to stimulate the proliferation of CD4⁺CD25⁺ T cells. DCs were isolated from draining skin lymph nodes and LCs isolated from epidermal sheets of wild type and K14-RANKL Tg mice and incubated with wild type CD4⁺CD25⁺ T cells at a ratio of T cells:APC = 1:2. After 4 days, T cell proliferation was measured via 3H-thymidine uptake. Data are shown as mean ± SD from three different experiments. * indicates statistical significance (Student's t-test; p < 0.05).
   d, Depletion of LCs in K14-RANKL Tg and wild type mice following topically treatment with mometason fuorate.
   e, LC depletion reduces the numbers of peripheral CD4⁺CD25⁺ T cells in wild type and K14-RANKL Tg mice. One week after treatment, lymph node and spleen cells were analyzed by flow cytometry for frequencies of CD4⁺CD25⁺ T cells. Data are shown as percentages of reduction in CD4⁺CD25⁺ T cell numbers in treated versus untreated mice. One out of three independent experiments is shown. * indicates statistical significance (Student's ttest; p < 0.05).

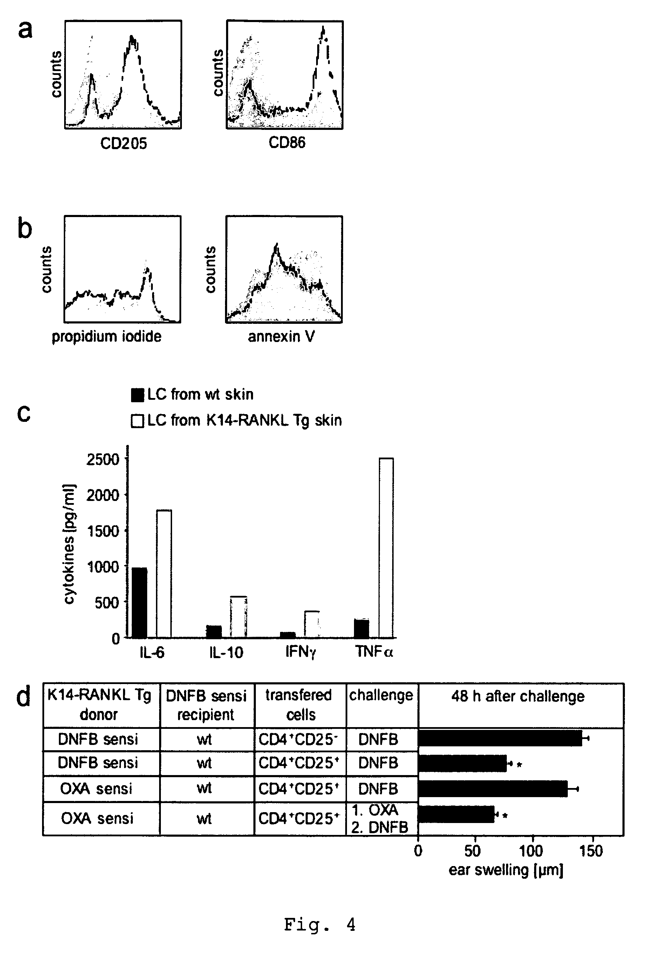

**Fig. 4.** Phenotypic and functional differences between Langerhans cells from wild type and K14-RANKL Tg mice.
   a, CD205 and CD86 expression on epidermal Langerhans cells. LCs from wt and K14-RANKL Tg epidermis were analyzed for surface marker expression by flow cytometry. Histograms from cells gated for Langerin expression are shown. Wild type LCs are shown in grey. LCs isolated from K14-RANK Tg are shown in black.
   b, Cell death. LCs from wt and K14-RANKL Tg epidermis were analyzed for spontaneous cell death using propidium iodide and annexin V staining. Cells were gated for CD11c. Wild type LCs are shown in grey. LCs isolated from K14-RANK Tg are shown in black.
   c, Enhanced cytokine production by LCs from K14-RANKL Tg epidermis. Spontaneous cytokine production of LCs was measured by cytometric bead assay inflammation kit. Data are shown as one out of three different experiments showing similar results.
   d, Adoptive transfer of CD4⁺CD25⁺ regulatory T cells suppresses allergic CHS responses in host mice. Wild type hosts were sensitized with DNFB or oxazolone and ear challenged with DNFB. 24h before challenge, sensitized wild type mice were injected with 1x10⁶ T cells from K14-RANKL-Tg donors as indicated. 12h before challenge, transferred cells were activated by epicutaneous application of oxazolone. Data are shown as mean ear swelling ± SD and are representative of 21 mice in three independent experiments. * indicates statistical significance (Student's t-test; p < 0.05).

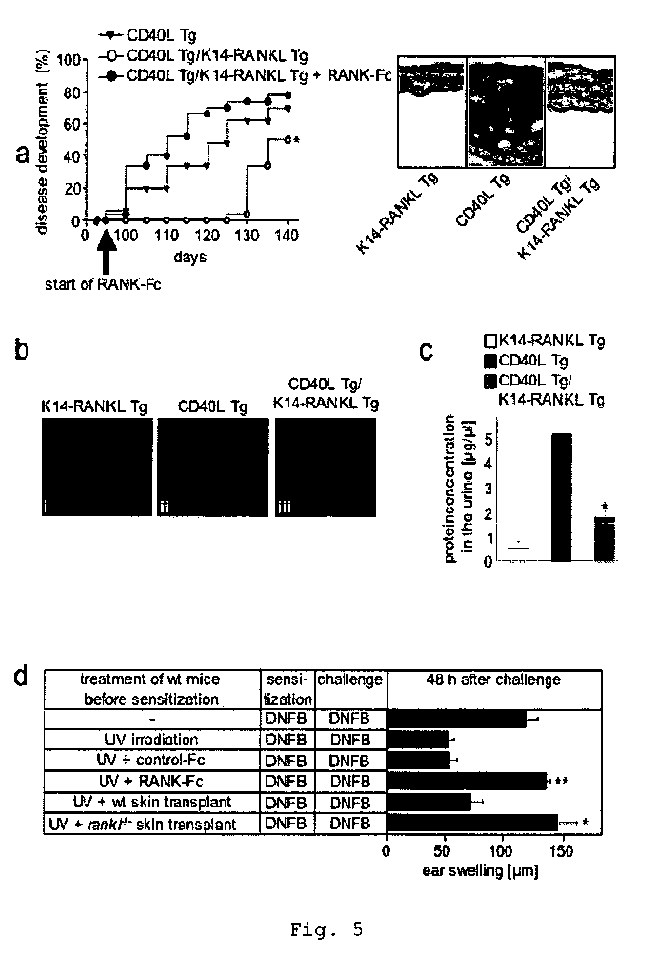

**Fig. 5.** RANKL over-expression in the skin suppresses allergic hypersensitivity responses and CD40L-driven systemic autoimmunity.
   a, Epidermal RANKL suppresses CD40L-induced systemic autoimmunity. CD40L Tg mice were crossed with K14-RANKL Tg mice and the onset of autoimmune dermatitis determined in the different experimental groups (Magnifications X 200; H&E staining of skin tissue). Treatment of CD40L/K14-RANKL double Tg mice with RANK-Fc abrogated the in vivo epidermal RANKL effect. Data were obtained from 10 mice for each group. * indicates statistical significance (log-rank test; p < 0.05).
   b, Epidermal RANKL suppressed development of antinuclear antibodies. Indirect fluorescent staining of HEp-2 cells incubated with serum from K14-RANKL Tg, CD40L Tg, and CD40L/K14-RANKL double Tg mice. (Magnifications X 200; serum dilution 1:80)
   c, Reduced proteinuria and rescued renal function by epidermal RANKL over-expression in CD40L Tg mice. Urine was harvested from autoimmune-prone CD40L Tg (n=10), K14-RANKL Tg (n=10), and CD40L/K14-RANKL double Tg (n=10) mice. Protein concentration was quantified using Bradford reagent. * indicates statistical significance (Student's t-test; p < 0.05).
   d, Blockade of RANKRANKL signalling prevents UV-induced suppression of contact hypersensitivity responses. Wild type mice (n = 6) were treated with control-Fc or RANK-Fc (10µg once per week for two weeks), UV irradiated on four consecutive days (100 mJ each day) and sensitized with DNFB via UV exposed skin. Additional groups of wt mice (n = 5) were transplanted with 5 cm² skin biopsies from wt or rankl^{-/-} mice. Four weeks later transplanted mice were UV irradiated and sensitized with DNFB on the grafted skin. Data are shown as mean ear swelling ± SD. Statistical significance was calculated by Student's t-test: * p < 0.05 vs. wild type skin transplant, ** p < 0.05 vs. injection of rat-IgG antibody.

### EXAMPLES:

### Materials and Methods:

### Generation of RANKL transgenic mice:

The gene for murine RANK ligand was placed under the control of the human Keratin-14 (K14) promoter (32). The K14 expression cassette included the K14 promoter, a rabbit β-globin intron, a BamH1 cloning site and the K14 polyadenylation site. The BamH1 cloning site was modified by ligating a polylinker into this site resulting in a multiple cloning site containing the restriction enzyme sites SalI, BglII, BamH1 and XbaI. The RANKL cDNA was flanked with SalI and BglII linkers by PCR using the primers AM47 (5'-TCCGTCGACGCCACCATGCGCCGGGCCAGCCGA-3' which includes the Kozak sequence GCCACC and AM48 5'-TCCTGATCAAG-ATCTTCAGTCTATGTCCTGAA-3' (MWG Biotech, Ebersberg, Germany). The amplification protocol used was 95°C for 3 min followed by cycles of 40: 95°C for 1 min; 52°C for 1 min; 72°C for 2 min x 30; 72°C for 5 min. As the RANKL cDNA has an internal restriction site for BglII, full length cDNA was obtained by partial digestion with SalI and BglII. The resulting fragment was then cloned into the SalI/BglII sites of the K14 expression cassette. Orientation of the insert was confirmed by restriction analysis and sequencing. Plasmid DNA used for microinjections was purified using the Jet Star Maxiprep Kit (Genomed, Bad Oeyenhausen, Germany). The insert was separated from the vector sequences by electrophoresis following digestion with XbaI and XhoI, extracted from the gel, resuspended in TE buffer (10 mM Tris pH 7.4; 1 mM EDTA), and used for microinjection at a concentration of 2 ng/µl into mouse C57BL/6/C3H/HeN F1 x C57BL/6 and FVB/N oocytes. Two founder lines with similar transgene expression were identified by PCR (AM28: 5'-CAATGATATACACTGTTTGAGATGA-3'; AM72: 5'-CATTGAT-GGTGAGGTGTGCAA-3'; cycling profile: 95°C for 3 min; [95°C for 1 min; 54°C for 1 min; 72°C for 1 min x 35; 72°C for 5 min] and Southern blotting. Experiments were performed with Tg mice on a C57BL/6/C3H-HeN background. CD40L Tg and rankl^{-/-} mice have been described previously (5,19). All mice were housed under specific pathogen-free (SPF) conditions and all experiments were performed according to institutional regulations.

### Skin transplantation:

Six-week-old wt mice were anesthetized with ketamine and xylazine. Skin transplantation was performed by excising a 5 cm² skin biopsy from the shaved back of recipient mice and replacing the skin with equal biopsies from sex and age matched wild type or rankl^{-/-} donor mice. Both wild type and rankl^{-/-} mice were on a C57Bl/6 background. Wounds were closed using Beriplast tissue adhesive (Aventis, Frankfurt, Germany) and transplants were monitored according to institutional guidelines. It should be noted that the transplants were successful in all cases as determined by macroscopic analysis, blood flow, and hair growth.

### Reverse Transcription PCR:

Mouse tissues were snap-frozen before RNA isolation and reverse transcription (RT). Groups of mice (n=5) were either irradiated on the shaved backs with 800 mJ/cm² UVB or epicutaneously infected with Herpes simplex virus type 1 as described (18). Subsequently, RNA was extracted from frozen tissues or the murine keratinocyte cell line pAM212 using RNeasy columns (Qiagen, Hilden, Germany) according to the manufacturer's instructions. cDNA was synthesized from 1 µg of total RNA using random hexa-nucleotide primers and the Reverse Transcription System (Promega, Madison, WI). Primers used were: RANKL forward, 5'-TCGCTCTGTTCCTGTACTTTCG -3' and RANKL reverse, 5'-GTAG-GTACGCTTCCCGATGT-3'; β-actin forward, 5'-GTGGGGCGCCCCAGGCACCA-3' and β-actin reverse, 5'-CTCCTTAATGTCACGCACGATTTC-3'; Cycling profile: 94°C for 4 min; [94°C for 1 min; 56°C for 1 min; 72°C for 1 min] x 40; 72°C for 5 min. Aliquots of PCR products were electrophoresed on 1.5% agarose gels and visualized by ethidium bromide staining.

### Immunohistochemistry and immunofluorescence:

Immunhistochemistry was performed on cryostat sections of human skin from different patients or epidermal ear sheets (6 - 8 µm) fixed in acetone according to standard methods (5). Ears were mechanically split into dorsal and ventral sides using forceps, incubated in 2 mM EDTA, washed with PBS and fixed in acetone. Slides were incubated in the appropriate dilutions of antibodies (anti-cytokeratin, anti-murine RANKL and anti-human-RANKL; all purchased from R&D Systems, Germany), or an isotype control and subsequently incubated with a horseradish peroxidase (HRP)-coupled, Oregon-Green- or Texas-Red labelled secondary antibody (Molecular Probes, Leiden, The Netherlands). Peroxidase activity was visualized using 3-amino-9-ethyl-carbazol as a chromogen. Tissues were counterstained with MAYER'S hemalaun solution (Merck, Darmstadt, Germany). For LC staining, epidermal sheets were blocked in 1 % FCS/PBS and stained with the antibody overnight (antimouse RANK, R&D Systems; anti-mouse I-A, clone M5/114, BD-PharMingen, Germany and anti-mouse CD207 (Langerin), clone 929F3, kindly provided by Dr. S. Saeland, Schering-Plough, Dardilly, France). Sheets were then incubated with an Oregon-Green- or TexasRed-coupled secondary antibody (Molecular Probes), mounted onto slides and examined using an Olympus BX61 microscope and the MetaMorph software (Visitron Systems, Germany). For removal of epidermal LCs, mice were topically treated on four consecutive days per week for four weeks with mometason furoat (Ecural®, Essex Pharma, Germany) and biopsied one week after the last treatment. One week after the last mometason furoat treatment, spleens and regional lymph nodes were removed and single cell suspensions prepared for flow cytometry.

### RANKL serum analysis:

RANKL protein levels were measured in serum obtained by cardiac puncture using commercial ELISA kits (R&D Systems) according to the manufacturer's instructions.

### Contact hypersensitivity (CHS) :

Mice were sensitized by painting 100 µl of 0.5 % DNFB, in acetone/olive oil (4/1) on the shaved back. For elicitation of CHS responses, 12 µl of 0.3 % DNFB were painted on both sides of left ear on day five. CHS was determined by the degree of ear swelling of the haptenexposed left ear compared to the ear thickness of the not-challenged right ear and measured with a micrometer (Mitutoyo, Japan) at indicated time points after challenge. Mice that were ear challenged without prior sensitization served as negative controls. To investigate Ag-specificity of CHS responses wild type and K14-RANKL Tg mice were DNFB sensitized and challenged with 10 µl 0.5% oxazolone. UV-induced suppression of contact hypersensitivity responses was performed by irradiating the mice on four consecutive days with 100 mJ/cm² each day on the shaved back For UV irradiation only of skin grafts surrounding skin areas were covered by topically applied zinc paste, which prevents UV penetration. On day five mice were sensitized with 100 µl of 0.5 % DNFB painted onto the irradiated skin. CHS responses were elicited by application of 12 µl of 0.3 % DNFB on both sides of the left ear five days after sensitisation.

### RANK-Fc blocking studies:

To inhibit RANK-RANKL interaction in vivo, mice were injected with RANK-Fc as previously described (3). Briefly, K14-RANKL Tg and wild type control mice were intravenously injected with 10 µg RANK-Fc once per week for 4 weeks beginning at 3 weeks of age. Control groups received 10µg rat IgG antibody (BD-Pharmingen, Germany). CD40L/K14-RANKL double Tg mice were treated with 10 µg RANK-Fc once per week for 6 weeks beginning at 16 weeks of age. In contact hypersensitivity experiments, wild type mice were injected intravenously with 10 µg RANK-Fc or rat IgG antibody once per week for two weeks beginning at 8 weeks of age.

### Systemic autoimmunity:

Development of autoimmunity was determined as described (5). Briefly, groups of CD40L Tg (n=10) and CD40L/K14-RANKL double Tg (n=10) were evaluated for onset of dermatitis three times per week by two independent investigators. Localisation, number and size of inflammatory lesions in the skin (dermatitis, red ears, macroscopic lesions on cheeks, snouts, head and neck, etc.) were documented. After 140 days mice from all groups were sacrificed and T cell subsets, antinuclear antibodies, histology of skin lesions as well as renal Ig depositions and proteinuria were analysed as described (5).

### Cell preparations and flow cytometry:

Single cell suspensions of spleens, lymph nodes, and thymi were prepared as described (5,18). For harvesting LCs, epidermal sheets were prepared by mechanically splitting mouse ears into dorsal and ventral sides using forceps. LCs were allowed to migrate out of the epidermis into culture medium for three days. Expression of cell surface and intracellular markers was analyzed by four-color flow cytometry on a FACScalibur™ cytometer (BD-PharMingen) using a CELLQuest™ software (BD PharMingen). Cells were stained in PBS containing 1% FCS using the following Abs from BD-Pharmingen: anti-CD205 (clone NLDC145), fluoresceine isothiocyanate-conjugated anti-CD45RB (clone 16A), anti-CD62L (clone Mel-14), anti-I-A (clone M5/114), anti-CD103 (clone 2E7), polyclonal goat anti-rat Ig, polyclonal goat anti-rabbit Ig, phycoerythrin-conjugated anti-CD25 (clone PC61), anti-CTLA-4 (clone UC10-4F10-11), anti-CD86 (clone GL1); peridinin chlorophyll protein-conjugated anti-CD4 (clone RM4-5), allophycocyan-in-conjugated anti-CD11c (clone HL3), anti-CD25 (clone PE61), mouse monoclonal Ab anti-neuropilin-1 (clone H-286; Santa Cruz Biotechnology, Santa Cruz, CA), anti-GITR (R&D Systems), fluoresceine isothiocyanat-labelled donkey antigoat Ig (Dianova, Hamburg, Germany), and Cy5-conjugated anti-CD207 (clone 929F3). Isotype-matched control antibodies were included in each staining. Apoptotic and necrotic cells were identified using an Annexin V apoptosis detection kit (BD-PharMingen) according to manufacturer's instructions.

### Adoptive transfers:

Donor mice were sensitized by painting 100 µl DNFB (0.5 % in acetone/olive oil, 4/1) or 100 µl oxazolone (2 % in acetone/olive oil, 4/1) on the shaved back on day 0. On day 5, spleens and regional lymph nodes were removed, single cell suspensions were prepared as described before, CD4⁺CD25⁻ and CD4⁺CD25⁺ cells were isolated by MACS (Miltenyi, Germany) and 1 x 10⁶ CD4⁺CD25⁻ or CD4⁺CD25⁺ T cells were injected i.v. into each recipient mouse. After 24 h, recipients were challenged by painting 12 µl 0.3 % DNFB or 12 µl 1 % oxazolone on both sides of the left ear and ear swelling was evaluated at the indicated time points.

### Proliferation assays:

CD4⁺CD25⁻ and CD4⁺CD25⁺ cells were sorted by MACS (Miltenyi) . CD4⁺CD25⁺ T cells (1x 10⁶/ml alone or mixed at indicated ratios) were cultured in triplicate 96-well-round-bottom plates and stimulated with 1 pg/ml anti-CD3 (clone 2c11) and 1 µg/ml anti-CD28 (clone 37.51; both BD-PharMingen). Proliferation assays were cultured in a final volume of 200 µl, 1 µCi/well ³H-thymidine was added for the last 12 h of the experiment, and thymidine incorporation was measured by scintillation counting. Recombinant murine IL-2 was purchased from R&D Systems and added to the assays at indicated concentrations. In some proliferation assays a transwell system with 0.3 µm pore size (BD Falcon, Germany) was used to evaluate the contact dependency of suppression.

### Cytometric bead array (CBA) :

The cytokine activity in culture supernatants of CD4⁺CD25⁺, CD4⁺CD25⁻, or Langerhans cells from K14-RANKL Tg and wt mice was assayed by CBA (BD PharMingen) according to the manufacturer's instructions. T cells (2 x 10⁶/ml) or Langerhans cells (1 x 10⁶/ml) were incubated for four days without any stimulation or with a combination of anti-CD3- and anti-CD28-antibodies (1 pg/ml each Ab) at 37°C and 5% CO₂ in 96-well round-bottom plates (BD Falcon, Germany) in a volume of 200 µl RPMI containing 10 % FCS. Supernatants were collected and subjected for cytokine quantification using CBA kits.

### Mixed lymphocyte reactions:

Allogeneic T cell (1 x 106/ml) were cultured in triplicates in 96-well-round-bottom plates, in a final volume of 200 µl, and dendritic cells isolated from regional lymph nodes by MACS using CD11c coupled magnetic beads or Langerhans cells were added at indicated ratios. Langerhans cells were allowed to migrate out of epidermal sheets. Mixed lymphocyte reactions were cultured for 96 h in a final volume of 200 µl. Proliferation of allogeneic T cells (H-2d) was assessed by 3H-thymidine incorporation (1 µCi/well) added for the last 12 h of the experiment.

### Thymectomy and thymus transplantation:

Three-day-old mice were anesthetized with a mixture of ketamine (Sanofi-Cerva, Germany) and xylazine (Sanofi-Cerva) at 20 µg/g body weight. Thymectomy was performed by aspiration of both thymic lobes through a small incision in the skin of wt or K14-RANKL Tg mice just above the sternum and successful thymectomy was confirmed at autopsy. The incision was closed using histoacryl tissue adhesive (Aesculap, Germany). Shamthymectomized mice underwent the same procedure, with the exception that the thymus was left intact. Thymus grafting was performed by placing two lobes of neonatal thymus under the left kidney capsule of thymectomized animals.

### Langerhans cell migration:

Migration of LC was monitored using fluoresceine isothiocyanat (FITC) as a tracer. Mouse ears of wt and K14-RANKL Tg mice were painted with 15 µl FITC (30 µg/µl in dibutylphtalate /aceton 1:1 supplemented with 5% DMSO, Sigma, Taufkirchen, Germany). Sixteen hours after treatment retroauricular lymph nodes were prepared and single cell suspensions analysed for CD207 (Langerin) expression.

### Example 1: Expression of RANKL in keratinocytes

It was analyzed whether RANKL is induced in keratinocytes in the skin following inflammation. Whereas normal skin keratinocytes did not express RANKL, inflammation of the skin due to UV exposure or Herpes simplex virus (HSV) infections resulted in RANKL expression (Fig. 1a). Moreover, RANKL expression was found in the murine keratinocyte cell line PAM212 (Fig. 1a). To investigate RANKL expression in human skin, biopsies from healthy volunteers, psoriasis, and lupus erythematosus patients were double-stained using RANKL and cytokeratin antibodies. The data in Fig. 1b show that RANKL expression can be detected in basal and suprabasal keratinocytes of human skin. In psoriatic lesions strong RANKL expression was found in keratinocytes of all epidermal layers whereas no RANKL expression was detectable in inflammatory lesions of cutaneous lupus erythematosus (Fig. 1b). These unexpected data show that RANKL expression is upregulated in keratinocytes during inflammation and exposure to environmental stimuli.

### Example 2: Role of RANKL in the cutaneous immune response

To investigate the potential role of RANKL signalling in cutaneous immune responses, transgenic mice that over-express full length murine RANKL under the transcriptional control of the Keratin-14 (K14) promoter were generated (Fig. 1c). Two transgenic founder lines were established. All described results were similar in both lines. Mice homozygous for the transgene are fertile and show no apparent abnormalities. Correct expression of the transgene was confirmed by immunohistochemistry (Fig.1c) and PCR. RANKL protein is expressed in the basal keratinocytes of the epidermis of K14-RANKL Tg animals similar to the expression pattern found in human skin but was absent in the epidermis of wild type control mice. Overexpression of RANKL in the skin did not alter the skin structure and development of skin appendages. Since RANKL can be cleaved into a soluble form (1,3,4), it was analyzed whether K14 promoter-driven over-expression of RANKL would result in increased systemic RANKL levels. However, over-expression of RANKL in the skin did not result in increased serum levels of RANKL (Fig. 1d).

### Example 3: Effect of RANKL on inflammatory cutaneous contact hypersensitivity

Since K14-RANKL Tg mice showed no apparent alterations of the "healthy" skin, it was tested whether RANKL over-expression could affect inflammatory cutaneous contact hypersensitivity (CHS) responses. Upon 2,4-dinitrofluorobenzene (DNFB) immunization followed by a local challenge at the ear, wild type mice developed an allergic hypersensitivity response (Fig. 1e). Intriguingly, K14-RANKL Tg animals demonstrated a significantly decreased CHS response. In both wild type and K14-RANKL Tg mice, this CHS response was antigen specific (Fig. 1e). This finding is in stark contrast to our previous data that K14 promoter-driven over-expression of CD40L, the closest homologue to RANKL among the TNF superfamily (3,4), triggers numerous immunostimulatory effects_in the skin and markedly enhances CHS responses5. These data show that RANKL over-expression in keratinocytes results in inhibition of antigen-specific cutaneous immunity and abrogated allergic contact hypersensitivity.

### Example 4: Effect of RANKL on T cell expression

Since CHS responses are controlled by T cells (6), T cell numbers and T cell subpopulations in spleens and lymph nodes of K14-RANKL Tg mice were analyzed. The ratios, total numbers, and surface receptor expression/levels of CD4⁺ T helper, CD8⁺ cytotoxic T cells, and B cells in the spleen and lymph nodes were comparable between K14-RANKL Tg and control littermates. Interestingly, the spleen and lymph nodes of K14-RANKL Tg mice showed 2-3 fold increased numbers of CD4⁺CD25⁺ regulatory T cells compared to wild type controls (Fig. 2a). This increased number of CD4⁺CD25⁺ regulatory T cells in the K14-RANKL Tg mice was dependent on RANKL-mediated signalling since blockade of this pathway by RANK-Fc resulted in a reduction of CD4⁺CD25⁺ regulatory T cells to normal numbers. Treatment with RANK-Fc also reduced the numbers of CD4⁺CD25⁺ regulatory T cells about 20-30% from normal levels in wild type mice. Furthermore, rankl^{-/-} mice showed markedly reduced numbers of splenic CD4⁺CD25⁺ T cells compared to their littermate controls (Fig. 2a). Thus, RANKL controls the numbers of CD4⁺CD25⁺ regulatory T cells. CD4⁺CD25⁺ T cells develop in the thymus and K14 expression has been described not only on basal keratinocytes but also on medullary thymic epithelial cells (7,8). Immunohistochemical and Western blot analysis of RANKL showed similar expression patterns in Tg thymus compared to wild type thymus specimens and similar numbers of CD4⁺CD25⁺ T cells were detectable in the thymus of K14-RANKL Tg and wild type mice. Moreover, while splenic CD4⁺CD25⁺ T cells are reduced in rankl^{-/-} mice, these knock-out mice exhibited normal numbers of thymic CD4⁺CD25⁺ T cells (wild type 2.65% n=10; rankl^{+/-} 2.31% n=5; rankl^{-/-}: 2.91% n=5, of total thymocytes). Nonetheless, it was possible that RANKL expression on thymic epithelial cells was responsible for the increased numbers of CD25⁺CD4⁺ T cells. To test this possibility, thymectomized K14-RANKL Tg mice were grafted with wild type thymus and thymectomized wild type mice transplanted with thymic tissue from K14-RANKL Tg mice (Fig. 2a). Eight to ten weeks after transplantation, lymph node and splenic numbers of CD4⁺CD25⁺ T cells were evaluated. Wild type mice transplanted with a thymus from K14-RANKL Tg mice developed normal numbers of CD4⁺CD25⁺ T cells. Again, increased numbers of CD4⁺CD25⁺ T cells were present in K14-RANKL Tg mice grafted with a wild type thymus (Fig. 2a). Thus, RANK-RANKL interactions appear to be relevant for the maintenance and/or peripheral expansion rather than the thymic development of CD4⁺CD25⁺ regulatory T cells.

Next, it was analysed whether the increased CD4⁺CD25⁺ population in K14-RANKL Tg mice expresses markers and displays functional properties that are characteristic for regulatory T cells. CD4⁺CD25⁺ regulatory T cells from K14-RANKL Tg mice indeed expressed prototypic surface markers such as CD45RBlow, neurophilin-1 (Nrp-1), intracellular CTLA-4, or integrin aEb7 (CD103) (Fig. 2b) (7,9-11). CD4⁺CD25⁺ T cells from K14-RANKL Tg and wild type mice expressed similar levels of Foxp3 as evidenced by quantitative real-time PCR. Similarly, in all thymic transplantion experiments (Fig. 2a), the resulting peripheral CD4⁺CD25⁺ T cells expressed markers characteristic for regulatory T cells, i.e. CD45RBlow, Nrp-1, and CTLA-4. CD4⁺CD25⁺ regulatory T cells are unable to proliferate in response to mitogenic Abs reactive to the T cell receptor complex7,12. Accordingly, CD4⁺CD25⁺ T cells from K14-RANKL Tg mice failed to proliferate upon stimulation with soluble anti-CD3/CD28 Abs (Fig. 2c). This anergic state could be overcome by adding IL-2.

CD4⁺CD25⁺ T cells from K14-RANKL Tg mice produced similar levels of IL-2, IL-4, IL-6, TNF-α, as well as IFN-γ compared to CD4⁺CD25⁺ T cells from wild type mice.

CD4⁺CD25⁺ T cells from both wild type and K14-RANKL Tg mice produced IL-10 upon stimulation (Fig. 2c).

In humans and mice, CD4⁺CD25⁺ regulatory T cells play an important role for immune tolerance by suppressing self-reactive T cells (7,13,14). To test whether CD4⁺CD25⁺ T cells from K14-RANKL Tg mice are indeed functionally suppressive, CD4⁺CD25⁺ regulatory T cells from wild type and K14-RANKL Tg mice was co-incubated with CD4⁺CD25⁻ T cells from either wild type or K14-RANKL Tg mice. Upon stimulation with anti-CD3 plus anti-CD28, the CD4⁺CD25⁺ T cells from K14-RANKL Tg and wild type mice suppressed the proliferation of wild type CD4⁺CD25⁻ T cells to a similar extend (Fig. 2d). Moreover, CD4⁺CD25⁻ T cells isolated from K14-RANKL Tg mice could be suppressed by wild type or K14-RANKL Tg regulatory T cells indicating that RANKL expression in the skin does not change the sensitivity of CD4⁺CD25⁻ T cells to be suppressed by regulatory T cells. Trans-well experiments were performed to demonstrate the need for contact-dependent suppression of CD4⁺CD25⁺ T cells from K14-RANKL Tg mice. Suppressor function was lost in vitro when CD4⁺ T cell subsets had no contact to each other. Thus, expression of RANKL in keratinocytes results in the systemic expansion of CD4⁺CD25⁺ T cells that show phenotypic and functional characteristics of regulatory T cells.

### Example 5: Influence of the local expression of RANKL in

### the skin on the numbers of regulatory T cells in lymphoid tissues

RANKL over-expression in the epidermis does not change systemic levels of soluble RANKL (Fig. 1d) and expression of the RANKL receptor RANK was not detectable on CD4⁺CD25⁺ T cells. Besides macrophages and osteoclasts, dendritic cell subsets constitutively express the RANKL receptor RANK (1,4,15). Thus, it was tested whether Langerhans cells (LC), the resident dendritic cells in the epidermis, express RANK. Indeed, epidermal LCs express RANK protein (Fig. 3a).

Under steady state conditions, LCs continuously migrate from the skin to the draining lymph nodes and play an important role in the induction of antigen-specific T cell activation (16,17). The numbers of epidermal LCs and the migratory capacity of antigen-laden LCs to lymph nodes in K14-RANKL Tg and control littermate mice were analysed. Using immunohistochemistry to visualize MHC class II I-A and Langerin as molecular markers for LCs, normal numbers and distributions of epidermal LCs were found in K14-RANKL Tg compared to wild type mice (Fig. 3a). To study the migration behaviors of antigen loaded LCs into local lymph nodes, K14-RANKL Tg and control wild type mice were epicutaneously painted with the fluorescent hapten FITC. Again, similar numbers of FITC⁺/Langerin⁺ LC were detected in skin-draining lymph nodes of wild type (1.15 ± 0.330 of total lymph node cells) and K14-RANKL Tg (1.60 ± 1.07%) mice following FITC application. These results indicate that RANKL over-expression has no apparent affect on numbers, distribution, or the migratory behavior of LCs.

### Example 6: Influence of RANKL on the functions of LCs

Next, it was investigated whether RANKL expression in keratinocytes changes the functions of LCs, which then result in the peripheral expansion of CD4⁺CD25⁺ T cells. To directly investigate whether DCs from K14-RANKL Tg mice can expand CD4⁺CD25⁺ T cells, CD11c⁺ DCs from peripheral skin-draining lymph nodes of wild type and K14-RANKL Tg mice were added to CD4⁺CD25⁺ T cells from wild type mice. Interestingly, DC from K14-RANKL Tg mice induced significantly enhanced proliferation of CD4⁺CD25⁺ T cells compared to DCs from wild type controls (Fig. 3b). Subsequently, LCs were prepared from epidermal sheets of wild type and K14-RANKL Tg mice. Similar to lymph node DCs, epidermal LCs from K14-RANKL Tg skin displayed an increased capacity to induce proliferation of CD4⁺CD25⁺ T cells (Fig. 3c). Proliferation of CD4⁺CD25⁺ T cells was dependent on DC-T cell contact. These data show that lymph node-derived DCs and epidermal LCs from K14-RANKL Tg mice induce increased proliferation of CD4⁺CD25⁺ T cells.

### Example 7: Involvement of LCs in regulating the number of peripheral T cells

To test whether LCs are indeed involved in regulating the numbers of peripheral CD4⁺CD25⁺ T cells in vivo, epidermal LCs were depleted from K14-RANKL Tg and wild type epidermis by topical treatment with mometason fuorate (18). Application of topical mometason fuorate depletes the epidermis from LCs for approximately two weeks but does not affect DC populations in the draining lymph nodes and does not alter K14-driven transgene expression (18). Subsequent to epidermal LC depletion (Fig. 3d), the frequencies of CD4⁺CD25⁺ T cells was analyzed. LC-depletion induced a significant 17-25% reduction of peripheral CD4⁺CD25⁺ T cell numbers in wild type mice (Fig. 3e). This reduction in CD4⁺CD25⁺ T cells was markedly increased in K14-RANKL Tg mice following LC-depletion (Fig. 3e). By contrast the total and relative numbers of CD4⁺CD25⁻ and CD3⁺CD8⁺ T cell populations in wild type as well as K14-RANKL Tg mice were not affected by topical mometason fuorate treatment. These findings indicate that epidermal LCs can modulate the peripheral homeostasis of regulatory CD4⁺CD25⁺ T cells.

### Example 8: Phenotypic and functional differences between Langerhans cells from wild type and K14-RANKL Tg mice.

Next, cell surface marker expression of epidermal LCs was analyzed to reveal potential differences in LC phenotypes. LCs isolated from wild type and K14-RANKL Tg epidermis showed similar expression of MHC class II (I-A), Langerin, and CD80 (Fig. 3a). Furthermore, no increased numbers of Langerin⁺ DCs were found in skin draining lymph nodes, showing that the high number of CD4⁺CD25⁺ regulatory T cells in K14-RANKL Tg mice was not a consequence of enhanced LC turnover. Importantly, LCs from K14-RANKL Tg mice show increased expression of CD205 (DEC205) and CD86 compared to controls (Fig. 4a). CD205 expression has been previously associated with DC mediated induction of CD4⁺CD25⁺ regulatory T cells (19) and CD86 has been implicated in the protection from spontaneous autoimmunity (20). Moreover, among LCs emigrating from Tg epidermal sheets, less apoptotic cells were detected compared to control LCs supporting that RANK-RANKL signaling prolongs the longevity of DC (Fig. 4b). Importantly, LCs from wild type and RANKL Tg skin also differed in their cytokine secretion profiles for TNF-α, IL-6, IL-10, and IFN-γ (Fig. 4c). Thus, LCs show normal distribution, normal MHC class II, RANK, and Langerin expression, and normal numbers in the skin of K14-RANKL Tg mice. However, exposure of LCs to epidermal RANKL results in LCs that display less apoptosis, increased cytokine production, and altered expression of surface receptors previously associated with immunosuppressive DC functions. Importantly, LCs from K14-RANKL Tg mice have an enhanced capacity to expand CD4⁺CD25⁺ regulatory T cells in vitro and in vivo. RANKL over-expression in the epidermis increased CD4⁺CD25⁺ regulatory T cell numbers, which shows a molecular mechanism by which cutaneous immune responses are down-regulated. Therefore it was investigated whether these cells suppress antigen-specific immune responses in vivo. First it was studied whether adoptive transfer of regulatory T cells results in the suppression of allergic contact hypersensitivity (CHS) in the skin. CD4⁺CD25⁻ T cells from DNFB sensitized K14-RANKL Tg mice were intravenously injected into DNFB sensitized wild type recipient mice. As expected these recipients were able to mount a strong CHS response upon DNFB challenge (Fig. 4d). In contrast, adoptive transfer of CD4⁺CD25⁺ T cells from DNFB sensitized K14-RANKL Tg mice into DNFB sensitized wild type mice significantly suppressed CHS responses after DNFB challenge.

Similarly, DNFB-sensitized wild type regulatory T cells strongly suppress CHS responses in recipient mice. It has been reported that antigen-specific activation of CD4⁺CD25⁺ regulatory T cells can suppress immune responses in an antigen non-specific fashion (7,12). To directly address this, CD4⁺CD25⁺ T cells from oxazolone sensitized K14-RANKL Tg mice into DNFB primed wild type recipients was injected (Fig. 4d). Upon DNFB challenge, normal CHS responses were measured supporting the concept that CD4⁺CD25⁺ regulatory T cells have to be activated by a specific antigen. In addition, CD4⁺CD25⁺ T cells from oxazolone sensitized K14-RANKL Tg mice were injected into DNFB primed recipients. In this experimental scenario, however, recipient mice were epicutaneously painted with oxazolone prior to DNFB ear challenge. This treatment regimen strongly suppressed CHS responses in the recipient mice (Fig. 4d). These findings indicate that once activated by a specific antigen, CD4⁺CD25⁺ T cells from K14-RANKL Tg mice can suppress CHS responses in an antigen non-specific way.

### Example 9: RANKL over-expression in the skin suppresses allergic hypersensitivity responses and CD40L-driven systemic autoimmunity.

Next it was tested whether CD4⁺CD25⁺ T cells from K14-RANKL Tg mice could also suppress the development of systemic autoimmunity induced by epidermal CD40L over-expression5, that is, whether RANKL in keratinocytes can override the action of CD40L to trigger systemic autoimmunity. Therefore autoimmune-prone CD40L Tg with K14-RANKL Tg mice to obtain double mutant mice was crossed. As described previously (5), CD40L single Tg mice develop a systemic autoimmune disease including scleroderma-like dermatitis, antinuclear antibodies, nephritis, and proteinuria (5). However, K14-RANKL/CD40L double Tg mice showed a significantly reduced and delayed onset of autoimmune dermatitis and weight loss (Fig. 5a). Analysis of CD8⁺ T cells in K14-RANKL/CD40L double Tg mice also revealed strongly reduced numbers of activated CD8⁺ T cells, suggesting that RANKL signalling suppresses differentiation of CD8⁺ T cells into cytotoxic effectors.

Intriguingly, all manifestations of autoimmunity in CD40L Tg mice, that is, the development of dermatitis, antinuclear antibodies, nephritis and proteinuria were inhibited in K14-RANKL/CD40L double Tg mice (Fig. 5a-c). Treatment of double Tg mice with RANK-Fc to block RANK-RANKL signalling, reversed the protective effect of epidermal RANKL expression (Fig. 5a). These data show that RANKL expression in the skin can suppress local cutaneous hyperallergic responses as well as CD40L driven systemic autoimmunity.

The skin represents an organ where interaction with the environment frequently stimulates the immune system. On the other hand, it has long been known that UV exposure or skin inflammation can result in immunosuppression. Moreover, recently it has been shown that epicutaneous immunization with autoantigenic peptides was able to prevent experimental allergic encephalitis (21) and that phenotypically immature LC, known to trigger regulatory T cells, chronically drain from inflamed skin to local lymph nodes in mice and humans (22,23). Thus, the quality of the inflammation in the skin, i.e., the up-regulation of certain molecules on keratinocytes, appears to dictate the outcome of the immune response. The critical molecules for skin-regulated immune homeostasis have not been known. Since UV irradiation up-regulates RANKL in skin (Fig. 1b) cutaneous RANKL is the missing link that couples UV radiation to immunosuppression. This was tested in a previously established model of UV-mediated suppression of CHS responses (24). Interestingly, injections of RANK-Fc into irradiated wild type mice resulted in protection against UV induced immunosuppression suggesting that RANK-RANKL interactions mediate the UV effects (Fig. 5d). To demonstrate that UV-induced immunosuppression is mediated via upregulation of cutaneous RANKL expression, wild type mice were transplanted with either wild type skin or rankl^{-/-} skin. After transplantation, mice were UV irradiated as well as sensitized via the grafted skin tissue. Intriguingly, wild type mice transplanted with rankl^{-/-} but not wild type skin were protected against UV-induced immunosuppression as indicated by normal CHS responses (Fig. 5d). These findings show that UV irradiation can up-regulate cutaneous RANKL expression and, most importantly, that RANKL mediates UV-induced immunsuppression.

RANKL and RANK are essential regulators of osteoclast differentiation and control the formation of a lactating mammary gland in pregnancy (1,2,25). In addition, RANKL expression is induced on activated T cells and RANK expression can be found on DCs (1,3). It has been reported that RANKL might be important to activate intestinal DCs and inhibition of RANKL-RANK resulted in reduced colitis (26). On the other hand it has been shown that RANK-Fc treatment can exacerbate disease in an inflammation-mediated Tg model for diabetes and decreases the numbers of CD4⁺CD25⁺ regulatory T cells in pancreas associated tissue (27). However, in the same experiment, inhibition of CD40L/CD40 interactions had the same effect as RANKL/RANK blockade (27). The data provided herein show for the first time that RANKL expression is inducible on keratinocytes and that this is a molecular pathway that couples the epidermis to local and systemic immunosuppression. Intriguingly, one of the strongest inducers of RANKL expression is Vitamin D₃ which is generated in the skin and has been long known to be immunosuppressive1. For instance, topical Vitamin D₃ derivatives are successfully used to treat psoriasis (28). Moreover, dexamethasone and Vitamin D₃ treatment of T cells can induce regulatory T cells (29).

### References:

1. Theill, L.E., et al. Annu. Rev. Immunol. 20:795-823 (2002).
2. Fata, J.E. et al. Cell 103:41-50 (2000).
3. Wong, B.R. et al. J. Biol. Chem. 272: 25190-25194 (1997).
4. Anderson, D.M. et al. Nature 390:175-179 (1997).
5. Mehling A. et al. J. Exp. Med. 194: 615-628 (2001).
6. Grabbe, S. & Schwarz, T. Immunol. Today 19:37-44 (1998).
7. Sakaguchi, S. et al. Immunol. Rev.182:18-32 (2001).
8. Laufer, T.M., et al. Nature 383: 81-85 (1996).
9. Read, S., et al. J. Exp. Med. 192:295-302 (2000).
10. Lehmann, J. et al. Proc. Natl Acad. Sci. U S A. 99:13031-13036 (2002).
11. Bruder, D. et al. Eur. J. Immunol. 34:623-630 (2004).
12. Shevach, E.M. Nat. Rev. Immunol. 2:389-400 (2002).
13. Viglietta, V., J. Exp. Med. 199: 971-979 (2004).
14. Cavani, A., et al. J. Immunol. 171: 5760-8 (2003).
15. Cremer, I. et al. Blood 100: 3646-3655 (2002).
16. Banchereau, J. & Steinman, R.M. Nature 392:245-52 (1998).
17. Steinman, R.M., et al. Annu. Rev. Immunol. 21: 685-711 (2003).
18. Loser, K. et al. Eur. J. Immunol. 34: 2022-2031 (2004).
19. Hawiger, D. et al. J. Exp. Med. 194: 769-779 (2001).
20. Salomon, B. et al. J. Exp, Med.194: 677-684 (2001).
21. Bynoe, M.S., et al. Immunity 19: 317-28 (2003).
22. Geissmann, F. et al. J. Exp. Med. 196: 417-430 (2002).
23. Scheinecker, C., J. Exp. Med. 196: 1079-1090 (2002).
24. Schwarz, A., et al. J. Immunol. 172: 1036-1043 (2004).
25. Kong, Y.Y. et al. Nature 397: 315-323 (1999).
26. Ashcroft, A.J. et al. Immunity 19: 849-861 (2003).
27. Green, E.A., et al. Immunity 16: 183-191 (2002).
28. Kragballe, K. J. Cell. Biochem. 49:46-52 (1992).
29. Barrat, F.J. et al. J. Exp. Med.195:603-616 (2002).
30. Leung, D.Y., et al. J. Clin. Invest. 113:651-657 (2004).
31. Kuhn, A., et al. J. Am. Acad. Dermatol. 45: 86-95 (2001).
32. Vassar, R., et al. Proc. Natl Acad. Sci. U S A. 86:1563-1567 (1989).

## Claims

1. Use of a compound with receptor activator of nuclear factor-κB ligand (RANKL) activity for the manufacture of a topical pharmaceutical formulation for the treatment or the prevention of inflammatory diseases.

2. Use according to claim 1, **characterised in that** the inflammatory diseases are local or systemic inflammations.

3. Use according to claim 1 or 2, **characterised in that** the inflammatory diseases are allergies, in particluar contact allergies, and/or autoimmune diseases.

4. Use according to claim 1 to 3, **characterised in that** the inflammatory diseases are viral inflammations, bacterial inflammations or inflammations caused by radiation, in particular by UV radiation orby exposure to irritants.

5. Use according to any one of claims 1 to 4, **characterised in** the inflammatory diseases are skin-associated diseases.

6. Use according to claim 5, **characterised in that** the skin-associated disease is selected from the group consisting of psoriasis, atopic dermatitis, alopecia areata, alopecia totalis, alopecia subtotalis, alopecia universalis, alopecia diffusa, lichen planus, dermatomyostis of the skin, atopic eczema, morphea, sklerodermia, psoriasis vulgaris, psoriasis capitis, psoriasis guttata, psoriasis inversa, alopecia areata ophiasis-type, androgenetic alopecia, allergic contact eczema, irritative contact eczema, contact eczema, pemphigus vulgaris, pemphigus foliaceus, pemphigus vegetans, scarring mucosal pemphigoid, bullous pemphgoid, mucous pemphigoid, dermatitis, dermatitis herpetiformis duhring, urticaria, necrobiosis lipoidica, erythema nodosum, lichen vidal, prurigo simplex, prurigo nodularis, prurigo acuta, linear IgA dermatosis, polymorphic light dermatoses, erythema solaris, lichen sclerosus et atrophicans, exanthema of the skin, drug exanthema, purpura chronica progressiva, dihidrotic eczema, Eczema, fixed drug exanthema, photoallergic skin reaction, lichen, simplex eriorale, dermatitis, "Graft versus Host-Disease", acne, rosacea, abnormal scarring, keloids, actinic keratosis, hyperkeratosis, epidermolytic hyperkeratosis, hyperkeratosis lenticularis perstans, keratosis pilaris, ichthyoses and vitiligo.

7. Use according to any one of claims 1 to 4, **characterised in that** the inflammatory diseases are selected from the group consisting of rheumatoid athritis, multiple sclerosis, type I diabetes, Hashimoto's disease, myokarditis, atherosclerosis, glomerulonephritis, autoimmune hepatitis and inflammatory Bowel disease.

8. Use according to any one of claims 1 to 7, **characterised in that** RANKL is recombinantly produced.

9. Use according to any one of claims 1 to 8, **characterised in that** the formulation is an ointment, a gel, a lotion, a foam, an emulsion, a liposome, a transferosome, a cream, a paste or a patch.

10. Use according to any one of claims 1 to 9, **characterised in that** the compound is comprised in the formulation in a concentration between 0,0001 to 1 % w/w, preferably between 0,001 to 0,5 % w/w, more preferably between 0,001 to 0,05 % w/w.

11. Use according to any one of claims 1 to 10, **characterised in that** the formulation further comprises at least one compound selected from the group consisting of cortisone, interleukins, in particular IL-1, IL-6 and IL-17, tumor necrosis factor α, prostaglandin E2 and vitamin D3.

12. Topical pharmaceutical formulation comprising receptor activator of nuclear factor-κB ligand (RANKL).

13. Formulation according to claim 12, **characterised in that** the formulation is an ointment, a gel, a lotion, a foam, an emulsion, a liposome, a transferosome, a cream, a paste or a patch.

14. Formulation according to claim 12 or 13, **characterised in that** the formulation comprises said compound in an amount of 0,0001 to 1 % w/w, preferably between 0,001 to 0,5 % w/w, more preferably between 0,001 to 0,05 % w/w.

15. Formulation according to any one of claims 12 to 14, **characterised in that** it further comprises at least one compound selected from the group consisting of cortisone, interleukins, in particular IL-1, IL-6 and IL-17, tumor necrosis factor α, prostaglandin E2 and vitamin D3.
